# EUROPEAN PATENT SPECIFICATION

(11) **EP 4 663 127 B1**
(45) Date of publication and mention of the grant of the patent: **26.08.2026**
(21) Application number: 25181930.6
(22) Date of filing: 11.06.2025
(51) Int. Cl.: A61B 6/03, A61B 6/00, G06N 3/0475, G06T 7/00, G16H 50/20, G06N 3/045, G06N 3/0455, G06N 3/047, G06N 3/08, G06N 3/084, G06N 3/088, G06N 3/094

(54) **SYSTEM AND METHOD FOR SUBJECT-SPECIFIC AMYLOID POSITRON EMISSION TOMOGRAPHY TRANSLATION USING CONDITIONED DIFFUSION-BASED GENERATIVE MODEL**
SYSTEM UND VERFAHREN ZUR SUBJEKTSPEZIFISCHEN AMYLOIDPOSITRONSEMISSIONSTOMOGRAFIEÜBERSETZUNG UNTER VERWENDUNG EINES KONDITIONIERTEN DIFFUSIONSBASIERTEN GENERATIVEN MODELLS
SYSTÈME ET PROCÉDÉ DE TRADUCTION PAR TOMOGRAPHIE PAR ÉMISSION DE LA POSITRON AMYLOÏDE SPÉCIFIQUE À UN SUJET À L'AIDE D'UN MODÈLE GÉNÉRATIF BASÉ SUR LA DIFFUSION CONDITIONNÉ

(30) Priority: 14.06.2024 IN 202421046160
(43) Date of publication of application: 17.12.2025
(73) Proprietor: Tata Consultancy Services Limited, Maharashtra (IN)
(72) Inventor: REDDY KANCHAM, Pavan Kumar, 560066 Bangalore, Karnataka (IN); GUBBI LAKSHMINARASIMHA, Jayavardhana Rama, 560066 Bangalore, Karnataka (IN); THOTTUPATTU, Alphin Jose, 500081 Hyderabad, Telangana (IN); PODUVAL, Murali, 400601 Mumbai, Maharashtra (IN); PAL, Arpan, 700091 Kolkata, West Bengal (IN)
(74) Representative: Goddar, Heinz J.

(56) References cited:
- US-A1- 2023 042 243
- DE VRIES BART MARIUS ET AL: "Classification of negative and positive 18F-florbetapir brain PET studies in subjective cognitive decline patients using a convolutional neural network", EUROPEAN JOURNAL OF NUCLEAR MEDICINE AND MOLECULAR IMAGING, vol. 48, no. 3, 2 September 2020 (2020-09-02), pages 721 - 728, XP037420659, ISSN: 1619-7070, DOI: 10.1007/S00259-020-05006-3
- THOTTUPATTU ALPHIN J ET AL: "Diffusion-Based Generative Model for subject-specific Amyloid Spread Prediction", PROCEEDINGS OF THE FIFTEENTH INDIAN CONFERENCE ON COMPUTER VISION GRAPHICS AND IMAGE PROCESSING, 13 December 2024 (2024-12-13), pages 1 - 7, XP093321549, ISBN: 979-8-4007-1075-9

## Description

### CROSS-REFERENCE TO RELATED APPLICATIONS AND PRIORITY

The present application claims priority to Indian application no. 202421046160 filed on June 14, 2024.

### TECHNICAL FIELD

The disclosure herein generally relates to the field of medical image processing, and, more particularly, to system and method for subject-specific amyloid positron emission tomography translation using conditioned diffusion-based generative model.

### BACKGROUND

Alzheimer's disease is a most common degenerative neurological condition. Research has shown that initiating treatment for Alzheimer's even before the onset of cognitive decline can effectively delay progression of symptoms. However, early detection is an important factor in ensuring effectiveness of medication, and it presents a significant challenge. Initiation of medication is contingent upon the confirmation of Alzheimer's disease staging. The widely accepted amyloid cascade hypothesis says that the Alzheimer's disease is characterized by the gradual accumulation of amyloid fibrils in the brain, occurring decades before the onset of noticeable symptoms. Consequently, amyloid is regarded as a valuable biomarker for detecting Alzheimer's disease. Fluid-based analysis of amyloid deposition is one of the commonly employed method. Moreover, assessing the spatial distribution of amyloid deposits is also an important consideration, providing additional insights to confirm the likelihood of future cognitive decline.

While amyloid positron emission tomography PET imaging is a primary method for directly assessing amyloid spatial distribution, its global utilization remains relatively limited. This limitation is due to the fact that amyloid deposition analysis primarily serves as a tool for early detection, rather than for monitoring disease progression, and is associated with significant cost. Accurate synthesis of amyloid positron emission tomography (PET) images from more commonly available Fluorodeoxyglucose (FDG) PET is required to be investigated with an aim of enhancing its availability for improved diagnosis. FDG PET is the most commonly used PET modality, widely employed to study glucose metabolism activity and to identify the presence of abnormalities through the assessment of disrupted glucose metabolism. Alteration of metabolism can impact both production and clearance of amyloid deposition. Insulin hormone is known to stimulate the uptake of glucose and plays a major role in glucose metabolism. Insulin resistance is known to stimulate Alzheimer's disease. Insulin resistance stimulates production of more insulin and dysfunction of Insulin-degrading enzyme (IDE) which is responsible for regulation of neuronal and microglial cell amyloid β-protein levels. This can be considered as an indirect connection between amyloid deposition and altered glucose metabolism. Studies also have shown that with increasing amyloid deposition and tau microfiber tangle formation, more microglial activation and hyper-metabolism occurs, which also relates FDG and Amyloid PET imaging. Due to these reasons, relationship between FDG and amyloid PET is investigated and more common FDG PET imaging is used to predict the amyloid PET. Alteration of glucose metabolism can be one among several factors and may not provide all the information needed for accurate prediction of amyloid. Amyloid deposition follows a noticeable pattern with four-five typical developmental stages in 80% of the subjects with Alzheimer's disease. The amyloid deposition trends across subjects are observed and relationship between amyloid deposition and glucose metabolism is explored.

Traditionally, multimodal image synthesis or conditional image synthesis plays an important role in field of medical image processing, as it aids in the identification and comprehension of disease progression. Within this context, the utilization of generative adversarial network (GAN)-based architectures, such as the conditional GAN (cGAN) network has demonstrated significant success. Nevertheless, one persistent challenge when employing GAN-based architectures is issue of mode collapse. This phenomenon occurs when the generator network fails to produce a wide range of diverse and meaningful outputs, resulting in limited variation and quality in the generated images. Diffusion models have shown promise in mitigating this problem. These models iteratively enhances images by introducing controlled noise at each step of the generation process. This strategic approach promotes creation of diverse and high-quality image samples, effectively addressing mode collapse limitations commonly encountered in conventional GAN-based architectures.

Specifically for subject-specific PET image synthesis only a few works exists. In one approach, three radio-tracer PET images were simultaneously trained using a star-GAN based architecture. This model was designed to predict any two of these PET images from the remaining one. Additionally, in another approach, FDG PET image was translated into amyloid PET image using an inspired network. First approach demands more modalities for optimal performance and the GAN based method exhibit a trade-off between performance and mode collapse, highlighting the need for a more advanced model for the amyloid PET image synthesis directly from FDG PET images. Document (DE VRIES BART MARIUS ET AL: "Classification of negative and positive 18F-florbetapir brain PET studies in subjective cognitive decline patients using a convolutional neural network", XP037420659) discloses Purpose: Visual reading of '¹⁸F-florbetapir positron emission tomography (PET) scans is used in the diagnostic process of patients with cognitive disorders for assessment of amyloid-8 (AB) depositions. However, this can be time-consuming, and difficult in case of borderline amyloid pathology. Computer-aided pattern recognition can be helpful in this process but needs to be validated. The aim of this work was to develop, train, validate and test a convolutional neural network (CNN) for discriminating between A&B negative and positive '¹⁸F-florbetapir PET scans in patients with subjective cognitive decline (SCD). Methods: '¹⁸F-florbetapir PET images were acquired and visually assessed. The SCD cohort consisted of 133 patients from the SCIENCe cohort and 22 patients from the ADNI database. From the SCIENCe cohort, standardized uptake value ratio (SUVR) images were computed. From the ADNI database, SUVR images were extracted. 2D CNNs (axial, coronal and sagittal) were built to capture features of the scans. The SCIENCe scans were randomly divided into training and validation set (5-fold cross-validation), and the ADNI scans were used as test set. Performance was evaluated based on average accuracy, sensitivity and specificity from the cross-validation. Next, the best performing CNN was evaluated on the test set. Results: The sagittal 2D-CNN classified the SCIENCe scans with the highest average accuracy of 99% +2 (SD), sensitivity of 97% +7 and specificity of 100%. The ADNI scans were classified with a 95% accuracy, 100% sensitivity and 92.3% specificity. Conclusion: The 2D-CNN algorithm can classify AB negative and positive '*F-florbetapir PET scans with high performance in SCD patients (ABSTRACT). Document (US 2023/042243 A1) discloses A system and method for predicting mild cognitive impairment (MCI) related diagnosis and prognosis utilizing hybrid machine learning. More specifically, the system and method produce predictions of MCI conversions to dementia and prognosis related thereof. Using available medical imaging and non-imaging data a diagnosis and prognosis model is trained using transfer learning. A platform may then receive a request from a clinician for a target patient's diagnosis or prognosis. The target patient's medical data is retrieved and used to create a model for the target patient. Then details of the target patient's model and the diagnosis and prognosis model are compared, a prediction is generated, and the prediction is returned to the clinician. As new medical data becomes available it is fed into the respective model to improve accuracy and update predictions (ABSTRACT).

### SUMMARY

Embodiments of the present disclosure present technological improvements as solutions to one or more of the above-mentioned technical problems recognized by the inventors in conventional systems. The invention is set out in appended set of claims.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 illustrates an exemplary system for subject-specific amyloid position emission tomography translation using conditioned diffusion-based generative model, according to some embodiments of the present disclosure.
FIG. 2 is a functional block diagram of architecture of conditioned diffusion-based generative model, using the system of FIG. 1, according to some embodiments of the present disclosure.
FIG. 3 illustrates an exemplary flow diagram illustrating a method for subject-specific amyloid position emission tomography translation using conditioned diffusion-based generative model, using the system of FIG. 1, in accordance with some embodiments of the present disclosure.
FIGS. 4A through 4C provide visual representations illustrating visual analysis of three sample predictions with the method for subject-specific amyloid positron emission tomography translation using conditioned diffusion-based generative model, in accordance with some embodiments of the present disclosure.
FIGS. 5A through 5C provide visual representations illustrating visual analysis of wrong predictions with the method for subject-specific amyloid positron emission tomography translation using conditioned diffusion-based generative model, in accordance with some embodiments of the present disclosure.
FIGS. 6A and 6B provides visual representations illustrating performance comparison between the method of the present disclosure with state of the art models and a baseline diffusion model for two subject sets, in accordance with some embodiments of the present disclosure.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments. It is intended that the following detailed description be considered as exemplary only, with the true scope being indicated by the following embodiments described herein.

Amyloid deposition is considered as a viable biomarker for Alzheimer's disease. It is an expensive and less used modality to study amyloid spatial distribution in brain. Embodiments of the present disclosure provide a system and method for subject-specific amyloid position emission tomography translation using conditioned diffusion-based generative model. The present disclosure discloses a deep learning network to synthesize amyloid PET images from more commonly used FDG-PET. In the method of the present disclosure, a connection between amyloid deposition and associated alteration in glucose metabolism is explored for synthesizing amyloid PET using a diffusion model inspired architecture.

Traditional diffusion models employ a Markovian chain, systematically introducing noise into an image in a stepwise manner until the image converges into Gaussian noise. Throughout this process, the model learns the intricate mapping between a normal distribution and target image distribution. A variant known as Diffusion Implicit Models which introduces complex dependencies across time-steps in the diffusion process, also exists. Conditional diffusion models have found applications in computer vision for tasks such as inpainting, segmentation, and colorization. Further, advantages of using *L*₂ loss over *L*₁ loss and self-attention mechanisms in diffusion models are investigated, resulting in generation of high-quality synthesized images. A recent attempt for image translation is by using stochastic Brownian Bridge process. However, such image translation works cannot be used in case of one-to-one mapping. In spite of using conditioning, one-to-one mapping is hard to achieve as they use random noise as input. Even-though segmentation models do one-to-one prediction, these methods are influenced by randomness in the input noise while translating one image into another.

The diffusion model inspired architecture of the present disclosure is designed to learn underlying amyloid deposition patterns with respect to age, gender and status of the disease. The diffusion model inspired architecture synthesizes high-quality images and outperforms existing baselines. When tested on classifier trained with real images for Alzheimer's detection, the synthesized images demonstrate only a marginal decrease in accuracy compared to the classifier tested with real images.

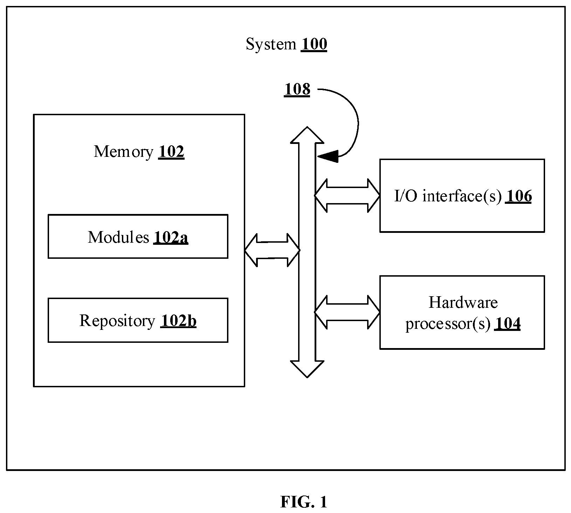

Referring now to the drawings, and more particularly to FIGS. 1 through 6B, where similar reference characters denote corresponding features consistently throughout the figures, there are shown preferred embodiments and these embodiments are described in the context of the following exemplary system and/or method.

FIG. 1 illustrates an exemplary system for auto repairing vulnerable code program fragments of a software according to some embodiments of the present disclosure. In an embodiment, the system 100 includes or is otherwise in communication with one or more hardware processors 104, communication interface device(s) or input/output (I/O) interface(s) 106, and one or more data storage devices or memory 102 operatively coupled to the one or more hardware processors 104. The one or more hardware processors 104, the memory 102, and the I/O interface(s) 106 may be coupled to a system bus 108 or a similar mechanism.

The I/O interface(s) 106 may include a variety of software and hardware interfaces, for example, a web interface, a graphical user interface, and the like. The I/O interface(s) 106 may include a variety of software and hardware interfaces, for example, interfaces for peripheral device(s), such as a keyboard, a mouse, an external memory, a plurality of sensor devices, a printer and the like. Further, the I/O interface(s) 106 may enable the system 100 to communicate with other devices, such as web servers and external databases.

The I/O interface(s) 106 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface(s) 106 may include one or more ports for connecting a number of computing systems with one another or to another server computer. Further, the I/O interface(s) 106 may include one or more ports for connecting a number of devices to one another or to another server.

The one or more hardware processors 104 may be implemented as one or more microprocessors, microcomputers, microcontrollers, digital signal processors, central processing units, state machines, logic circuitries, and/or any devices that manipulate signals based on operational instructions. Among other capabilities, the one or more hardware processors 104 are configured to fetch and execute computer-readable instructions stored in the memory 102. In the context of the present disclosure, the expressions 'processors' and 'hardware processors' may be used interchangeably. In an embodiment, the system 100 can be implemented in a variety of computing systems, such as laptop computers, portable computer, notebooks, hand-held devices, workstations, mainframe computers, servers, a network cloud and the like.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, the memory 102 includes a plurality of modules 102a and a repository 102b for storing data processed, received, and generated by one or more of the plurality of modules 102a. The plurality of modules 102a may include routines, programs, objects, components, data structures, and so on, which perform particular tasks or implement particular abstract data types.

The plurality of modules 102a may include programs or computer-readable instructions or coded instructions that supplement applications or functions performed by the system 100. The plurality of modules 102a may also be used as, signal processor(s), state machine(s), logic circuitries, and/or any other device or component that manipulates signals based on operational instructions. Further, the plurality of modules 102a can be used by hardware, by computer-readable instructions executed by the one or more hardware processors 104, or by a combination thereof. Further, the memory 102 may include information pertaining to input(s)/output(s) of each step performed by the processor(s) 104 of the system 100 and methods of the present disclosure.

The repository 102b may include a database or a data engine. Further, the repository 102b amongst other things, may serve as a database or includes a plurality of databases for storing the data that is processed, received, or generated as a result of the execution of the plurality of modules 102a. Although the repository 102b is shown internal to the system 100, it will be noted that, in alternate embodiments, the repository 102b can also be implemented external to the system 100, where the repository 102b may be stored within an external database (not shown in FIG. 1) communicatively coupled to the system 100. The data contained within such external database may be periodically updated. For example, new data may be added into the external database and/or existing data may be modified and/or non-useful data may be deleted from the external database. In one example, the data may be stored in an external system, such as a Lightweight Directory Access Protocol (LDAP) directory and a Relational Database Management System (RDBMS). In another embodiment, the data stored in the repository 102b may be distributed between the system 100 and the external database.

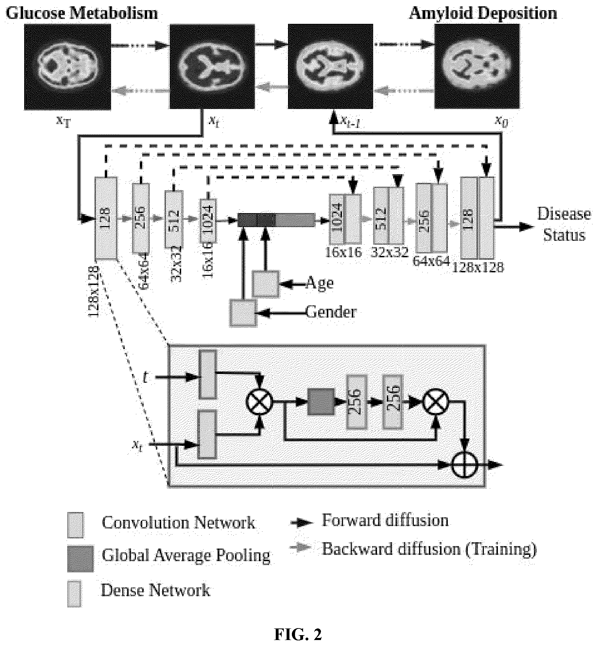

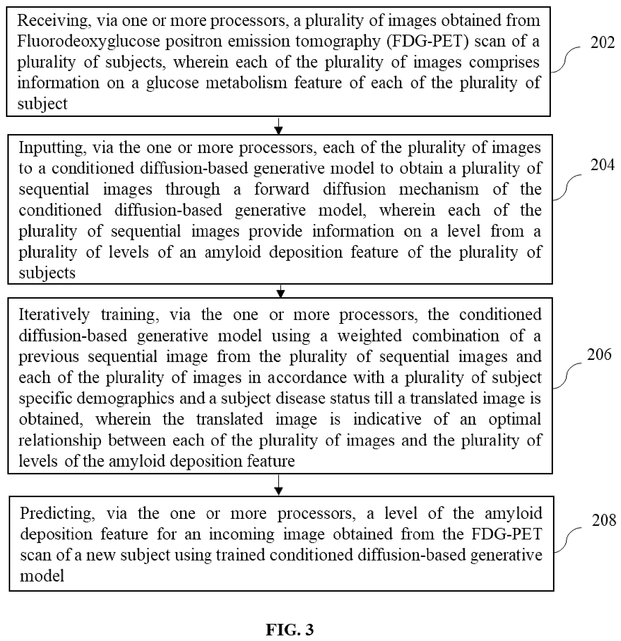

FIG. 2, with reference to FIG. 1, is a functional block diagram of architecture of conditioned diffusion-based generative model, using the system of FIG. 1, according to some embodiments of the present disclosure. FIG. 3 illustrates an exemplary flow diagram illustrating a method for subject-specific amyloid position emission tomography translation using conditioned diffusion-based generative model, using the system of FIG. 1, in accordance with some embodiments of the present disclosure.

Referring to FIG. 3, in an embodiment, the system(s) 100 comprises one or more data storage devices or the memory 102 operatively coupled to the one or more hardware processors 104 and is configured to store instructions for execution of steps of the method by the one or more processors 104. The steps of the method 200 of the present disclosure will now be explained with reference to components of the system 100 of FIG. 1, the block diagram of FIG. 2, the flow diagram as depicted in FIG. 3, and one or more examples. Although steps of the method 200 including process steps, method steps, techniques or the like may be described in a sequential order, such processes, methods and techniques may be configured to work in alternate orders. In other words, any sequence or order of steps that may be described does not necessarily indicate a requirement that the steps be performed in that order. The steps of processes described herein may be performed in any practical order. Further, some steps may be performed simultaneously, or some steps may be performed alone or independently.

In an embodiment, at step 202 of the present disclosure, the one or more hardware processors 104 are configured to receive a plurality of images obtained from Fluorodeoxyglucose positron emission tomography (FDG-PET) scan of a plurality of subjects. In an embodiment, the plurality of subjects include patients who are examined based on scan. Here, each of the plurality of images comprises information on a glucose metabolism feature of the each of the plurality of subjects.

Further, at step 204 of the present disclosure, the one or more hardware processors 104 are configured to input each of the plurality of images to a conditioned diffusion-based generative model to obtain a plurality of sequential images through a forward diffusion mechanism of the conditioned diffusion-based generative model. Each of the plurality of sequential images provide information on a level from a plurality of levels of an amyloid deposition feature of the plurality of subjects. In the present disclosure, Amyloid is synthesized by leveraging available information pertaining to Amyloid in FDG PET scans. There is reliance on connection between FDG and Amyloid PET imaging. Age and gender significantly impact amyloid levels and distinct distribution patterns exist between normal and cognitively impaired individuals. As shown in FIG. 2, the conditioned diffusion-based generative model is tailored to identify the optimal relationship between FDG PET imaging and amyloid levels. It leverages additional factors to aid the network in identifying trends through intrinsic grouping based on these patterns.

In an embodiment, in order to condition the FDG, instead of adding conditional information in layers, FDG is fed as the input to the conditioned diffusion-based generative model. The translation of images is acquired through a reversible diffusion process within the conditioned diffusion-based generative model. In the conditioned diffusion-based generative model, the forward diffusion mechanism is precisely defined to facilitate training of the conditioned diffusion-based generative model. In contrast to denoising models, where noise is introduced into the image data, in the conditioned diffusion-based generative model, in forward diffusion, the amyloid data distribution propagates into the FDG data distribution over time steps within the *0, T* time gap. For instance, the Amyloid distribution is considered as *p*(*x*₀) *=* 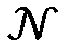 (*µ_{f}, σ_{f}*) and FDG distribution as *p*(*x_{T}*) *=* 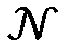 (*µₐ*, *σₐ*). The diffusion process is defined as a Markov chain with conditional probability of each state defined as *q*(*xₜ*/*x*_{*t*-1})*.* The diffusion at each state is controlled by a scheduled diffusion rate *β* with range [0, 1]. The forward diffusion mechanism is derived by gradually adding the FDG information to the amyloid data. In the diffusion process, at each step constant fraction $\frac{1}{T}$ of *x*₀ is erased, hence ${β}_{t} = \frac{1}{T - t + 1}$. Each step is ${x}_{t} = \sqrt{{α}_{t}} {x}_{0} + \sqrt{1 - {α}_{t}} {x}_{T}$, where ${α}_{t} = {\prod }_{0}^{t} 1 - {β}_{t}$*.* Hence, as shown in equation (1) below $q \left({x}_{t}\left|{x}_{t - 1}\right.\right) : N \left({x}_{t};{μ}_{f}+\sqrt{1 - {β}_{t}}\left({x}_{t - 1}-{μ}_{a}\right),{β}_{t}{σ}_{f}\right)$

Further, at step 206 of the present disclosure, the one or more hardware processors 104 are configured to iteratively train the conditioned diffusion-based generative model using a weighted combination of a previous sequential image from the plurality of sequential images and each of the plurality of images in accordance with a plurality of subject specific demographics and a subject disease status till a translated image is obtained. The plurality of subject specific demographics may include but are not limited to age factor, gender, genetic, lifestyle, environmental factor, and/or the like. The translated image is indicative of an optimal relationship between each of the plurality of images and the plurality of levels of the amyloid deposition feature. In an embodiment, the conditioned diffusion-based generative model is tailored to identify the optimal relationship between the FDG PET imaging and amyloid levels. The optimal relationship between each of the plurality of images and the plurality of levels of the amyloid deposition feature is indicative of a maximum level of disease progression and helps to monitor disease progression. As shown in FIG. 2, the conditioned diffusion-based generative model comprises an encoder network, a decoder network, and a multi-layer perceptron (MLP) layer in between the encoder network and the decoder network. The encoder network comprises a four-level encoder where each level comprises a single convolutional layer that uses a channel attention. At each convolutional layer, a time-step input encoded with a dense layer is provided to guide the encoder network in interpreting the time-step input. In an embodiment, the plurality of subject specific demographics are passed into the MLP layer of the conditioned diffusion-based generative model after encoding.

The step 206 is better understood by way of the following description provided as exemplary explanation.

The conditioned diffusion-based generative model (*f_{θ}*) is trained to predict Gaussian backward process parameterized by *θ*. The conditioned diffusion-based generative model is trained with the plurality of sequential images generated by the forward diffusion mechanism to predict and separate the FDG content at that a particular time instant. The *f_{θ}* models the backward diffusion as shown in equation (2) below: ${p}_{θ} \left({x}_{t - 1}\left|{x}_{t}\right.\right) : N \left({x}_{t - 1};{μ}_{θ}\left({x}_{t}, t\right),{Σ}_{θ}\left({x}_{t}, t\right)\right.$ As shown in FIG. 2, a U-Net based architecture with multi-layer perceptron (MLP) layer in between the encoder network and decoder network is used to train *f_{θ}.* The U-Net based architecture features a four-level encoder, each level comprising a single convolutional layer. The layer's feature count ranges from 128 to 1024 in a top-to-bottom progression. The convolutional layer uses a channel attention. At each convolutional layer, the time-step input encoded with a dense layer is provided to guide the network in interpreting the time-step, as illustrated in FIG. 2. The age and gender are also passed into the MLP layer of the conditioned diffusion-based generative model after encoding with dense layer.

At step 208 of the present disclosure, the one or more hardware processors 104 are configured to predict a level of the amyloid deposition feature for an incoming image obtained from the FDG-PET scan of a new subject using trained conditioned diffusion-based generative model. In an embodiment, the incoming image is a test image. Finally with an additional dense layer in the U-Net based architecture, the conditioned diffusion-based generative model predicts the disease status. Equally weighted Structural Similarity Index (SSIM), *L*₁, and *L*₂ losses are used in the model training. The conditioned diffusion-based generative model architecture helps to learn the smooth translation between the FDG and Amyloid PET images. The disease status prediction along with age and gender information forces the conditioned diffusion-based generative model to learn inherent grouping or amyloid deposition trends spanning across subjects. Using the learned model *f_{θ}*, the backward prediction is *x*_{*t*-1} *= µ_{θ}*(*xₜ, t*) *+* Σ*_{θ}*(*xₜ, t*)*x_{T}*, where Σ*_{θ}* is fixed in general.

### Experimental Results:

**Experimental Setting:** In the present disclosure, a state in the art dataset (i.e., Alzheimer's Disease Neuroimaging Initiative (ADNI) data set) is used for validation. F-18 fluorodeoxyglucose (FDG) and AV45 amyloid PET images associated with five distinct disease staging are used: Alzheimer's Disease (AD), Early Mild Cognitive Impairment (EMCI), Mild Cognitive Impairment (MCI), Late Mild Cognitive Impairment (LMCI), and Cognitively Normal (CN). In the present disclosure, axial central slices from registered volumes (128 ×128×128) are examined. Approximately 1700 images were used, allocating 80% for training and 20% for testing the models. The pixel intensities corresponds to normalized stands for standardized uptake value ratio. It is noted that the state in the art dataset (i.e., ADNI data set) has longitudinal data and it is ensured that follow-up scans of same individual were not included in both the training and test sets.

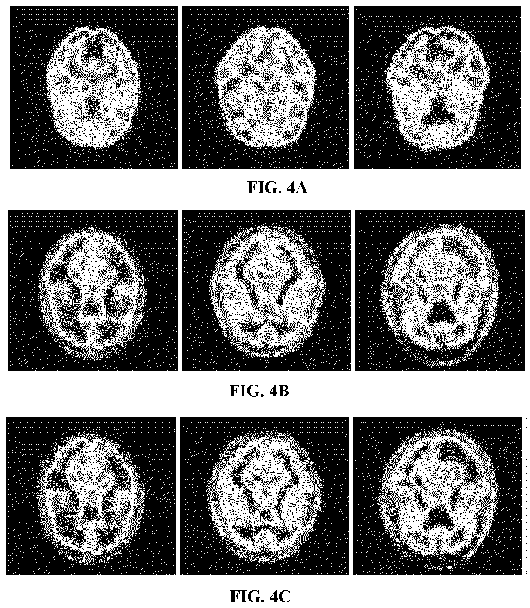

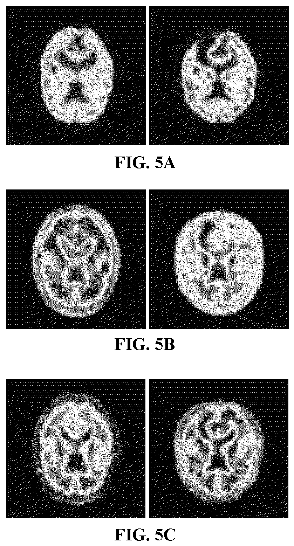

**Visual Assessment and comparative analysis:** FIGS. 4A through 4C provide visual representations illustrating visual analysis of three sample predictions with the method for subject-specific amyloid position emission tomography translation using conditioned diffusion-based generative model, in accordance with some embodiments of the present disclosure. FIGS. 4A through 4C represent visual representation of a FDG PET sample prediction, an amyloid PET sample prediction, and a predicted Amyloid PET sample prediction respectively. The visual similarity between FIG. 4B and FIG. 4C demonstrates the effectiveness of the method of the present disclosure. In FIGS. 4A through 4C, a few sample images generated by the method of the present disclosure are presented, including input, ground truth, and absolute error. The minimal difference between the predicted images and the ground truth underscores accuracy of the method of the present disclosure. While the method of the present disclosure consistently demonstrates high accuracy in predictions across majority of the cases, it is essential to confront and address instances of failure. FIGS. 5A through 5C provide visual representations illustrating visual analysis of wrong predictions with the method for subject-specific amyloid position emission tomography translation using conditioned diffusion-based generative model, in accordance with some embodiments of the present disclosure. These outliers underscore the challenges to further enhance the robustness of the method of the present disclosure. In an embodiment, effectiveness of the method of the present disclosure is evaluated using both Mean Squared Error (MSE) and Structural Similarity Index (SSIM) metrics, with results detailed in Table 1. Table 1 below provides a performance comparison between the method of the present disclosure with state of the art models such as cycle generative adversarial network (GAN), pix2pix and a baseline diffusion model where subject demographics are not used for training.

**Table 1**

| | MSE | SSIM |
|---|---|---|
| Cycle GAN | 0.008 ± 0.004 | 0.78 ± 0.10 |
| pix2pix | 0.006 ± 0.003 | 0.89 ± 0.08 |
| Baseline Diffusion model | 0.003 ± 0.010 | 0.89±0.07 |
| Method of the present disclosure | 0.002 ± 0.003 | 0.91 ± 0.09 |

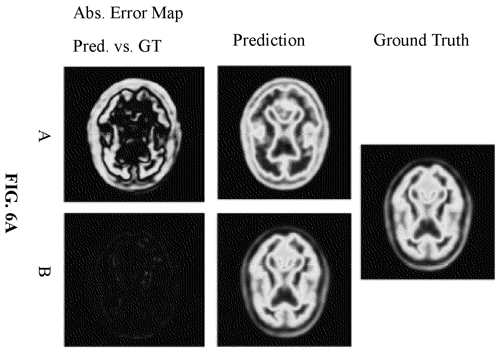

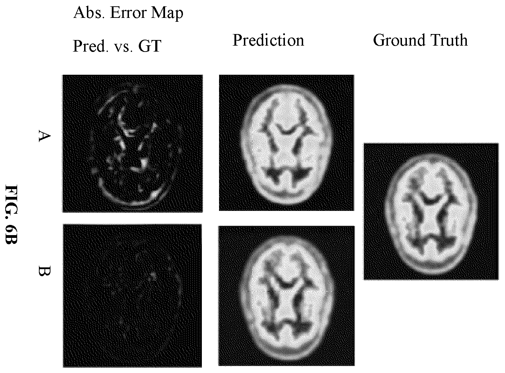

The baseline diffusion model, that uses the method of the present disclosure trained without age, gender or category was used. As shown in Table 1, the outcomes indicate superior alignment with the ground truth when the method of the present disclosure is applied to test set. FIGS. 6A and 6B provide visual representations illustrating performance comparison between the method of the present disclosure with state of the art models and a baseline diffusion model for two subject sets, in accordance with some embodiments of the present disclosure. Each set in FIGS. 6A and 6B represents an amyloid PET ground truth, predictions using respective method, and absolute error maps. Complementing the quantitative analysis, visual comparisons presented in FIGS. 6A and 6B reinforce robust performance of use of the method of the present disclosure.

**Validation:** To validate the method of the present disclosure, a classifier to detect Alzheimer's was created as a downstream application. The classifier is trained and tested on train and test sets used in previous experiment, involving only Alzheimer's Disease (AD) and normal classes from sets for classification. In this experiment, a convolutional neural network (CNN) with three convolutional layers, max-pooling, and two fully connected layers was employed. Performance evaluation was conducted using an independent set of real data (SET1) and corresponding synthesized data with conditioned diffusion-based generative model (SET2), baseline diffusion model (SET3) and pix2pix (SET4). Table 2 provides Alzheimer's detection accuracy using real Amyloid images (SET1), synthesized Amyloid images with the method of the present disclosure (SET2), synthesized Amyloid images with baseline diffusion model (SET3) and synthesized Amyloid images using pix2pix (SET4).

**Table 2**

| | SET1 | SET2 | SET3 | SET 4 |
|---|---|---|---|---|
| Classifier Accuracy | 93% | 85% | 73% | 72% |

As shown in Table 2, in relative terms, the synthesized images using the method of the present disclosure shows minimal reduction in accuracy (i.e., ≈ 8%) compared to real images. This suggests that the real and synthesized images with the method of the present disclosure are similar for the classifier, indirectly affirming the quality of the synthesized images. The qualitative and the quantitative results reflect the robustness of the method of the present disclosure. Further, the results of downstream task demonstrate usefulness of the method of the present disclosure in a real world setting. There is a well-defined correlation noticed in the results between the actual ground truth and the synthesized or predicted amyloid PET images. The spatial distribution of amyloid and the concentration of amyloid in the cerebral cortices and juxta ventricular areas closely resemble the ground truth thus validating the utility of such methodologies in easing the burden of frequent and more advanced scans in the diagnosis and prognosis of Alzheimer's disease.

The written description describes the subject matter herein to enable any person skilled in the art to make and use the embodiments.

Embodiments of the present disclosure provide a system and method for subject-specific amyloid positron emission tomography translation using conditioned diffusion-based generative model. The present disclosure discloses an architecture for synthesizing subject-specific amyloid image with diffusion model. In the present disclosure, first effectiveness of the relationship between FDG and amyloid PET images is identified. Further, a framework is provided to synthesize Amyloid PET by utilizing its connection to FDG PET of the same subject and the cross-subject trends in amyloid deposition by incorporating age, gender and disease status information in the learning process. In the other words, a diffusion model inspired image translation is provided to synthesize Amyloid PET from FDG PET and cross-subject amyloid deposition patterns.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated herein by the following claims.

## Claims

1. A processor implemented method (200), comprising:
receiving (202), via one or more processors, a plurality of images obtained from Fluorodeoxyglucose positron emission tomography, FDG-PET, scan of a plurality of subjects, wherein each of the plurality of images comprises information on a glucose metabolism feature of each of the plurality of subjects;
inputting (204), via the one or more processors, each of the plurality of images to a conditioned diffusion-based generative model to obtain a plurality of sequential images through a forward diffusion mechanism of the conditioned diffusion-based generative model, wherein each of the plurality of sequential images provide information on a level from a plurality of levels of an amyloid deposition feature of the plurality of subjects, wherein the conditioned diffusion-based generative model comprises an encoder network, a decoder network, and a multi-layer perceptron MLP, layer in between the encoder network and the decoder network, wherein the encoder network comprises a four-level encoder where each level comprises a single convolutional layer that uses a channel attention, and wherein at each convolutional layer, a time-step input encoded with a dense layer is provided to guide the encoder network in interpreting the time-step input;
iteratively training (206), via the one or more processors, the conditioned diffusion-based generative model using a weighted combination of a previous sequential image from the plurality of sequential images and each of the plurality of images in accordance with a plurality of subject specific demographics and a subject disease status till a translated image is obtained, wherein the translated image is indicative of an optimal relationship between each of the plurality of images and the plurality of levels of the amyloid deposition feature; and
predicting (208), via the one or more processors, a level of the amyloid deposition feature for an incoming image obtained from the FDG-PET scan of a new subject using trained conditioned diffusion-based generative model.

2. The processor implemented method as claimed in claim 1, wherein the plurality of subject specific demographics are passed into the MLP layer of the conditioned diffusion-based generative model after encoding.

3. A system (100), comprising:
a memory (102) storing instructions;
one or more communication interfaces (106); and
one or more hardware processors (104) coupled to the memory (102) via the one or more communication interfaces (106), wherein the one or more hardware processors (104) are configured by the instructions to:
receive a plurality of images obtained from Fluorodeoxyglucose positron emission tomography, FDG-PET, scan of a plurality of subjects, wherein each of the plurality of images comprises information on a glucose metabolism feature of each of the plurality of subjects;
input each of the plurality of images to a conditioned diffusion-based generative model to obtain a plurality of sequential images through a forward diffusion mechanism of the conditioned diffusion-based generative model, wherein each of the plurality of sequential images provide information on a level from a plurality of levels of an amyloid deposition feature of the plurality of subjects, wherein the conditioned diffusion-based generative model comprises an encoder network, a decoder network, and a multi-layer perceptron, MLP, layer in between the encoder network and the decoder network, wherein the encoder network comprises a four-level encoder where each level comprises a single convolutional layer that uses a channel attention, and wherein at each convolutional layer, a time-step input encoded with a dense layer is provided to guide the encoder network in interpreting the time-step input;
iteratively train the conditioned diffusion-based generative model using a weighted combination of a previous sequential image from the plurality of sequential images and each of the plurality of images in accordance with a plurality of subject specific demographics and a subject disease status till a translated image is obtained, wherein the translated image is indicative of an optimal relationship between each of the plurality of images and the plurality of levels of the amyloid deposition feature; and
predict a level of the amyloid deposition feature for an incoming image obtained from the FDG-PET scan of a new subject using trained conditioned diffusion-based generative model.

4. The system (100) as claimed in claim 3, wherein the plurality of subject specific demographics are passed into the MLP layer of the conditioned diffusion-based generative model after encoding.

5. One or more non-transitory machine-readable information storage mediums comprising one or more instructions which when executed by one or more hardware processors cause:
receiving a plurality of images obtained from Fluorodeoxyglucose positron emission tomography FDG-PET, scan of a plurality of subjects, wherein each of the plurality of images comprises information on a glucose metabolism feature of each of the plurality of subjects;
inputting each of the plurality of images to a conditioned diffusion-based generative model to obtain a plurality of sequential images through a forward diffusion mechanism of the conditioned diffusion-based generative model, wherein each of the plurality of sequential images provide information on a level from a plurality of levels of an amyloid deposition feature of the plurality of subjects, wherein the conditioned diffusion-based generative model comprises an encoder network, a decoder network, and a multi-layer perceptron MLP, layer in between the encoder network and the decoder network, wherein the encoder network comprises a four-level encoder where each level comprises a single convolutional layer that uses a channel attention, and wherein at each convolutional layer, a time-step input encoded with a dense layer is provided to guide the encoder network in interpreting the time-step input;
iteratively training the conditioned diffusion-based generative model using a weighted combination of a previous sequential image from the plurality of sequential images and each of the plurality of images in accordance with a plurality of subject specific demographics and a subject disease status till a translated image is obtained, wherein the translated image is indicative of an optimal relationship between each of the plurality of images and the plurality of levels of the amyloid deposition feature; and
predicting a level of the amyloid deposition feature for an incoming image obtained from the FDG-PET scan of a new subject using trained conditioned diffusion-based generative model.

6. The one or more non-transitory machine-readable information storage mediums as claimed in claim 5, wherein the plurality of subject specific demographics are passed into the MLP layer of the conditioned diffusion-based generative model after encoding.

## Patentansprüche

1. Prozessorimplementiertes Verfahren (200), umfassend:
Empfangen (202), über einen oder mehrere Prozessoren, einer Mehrzahl von Bildern, die aus einem Fluordeoxyglucose-Positronen-Emissionstomographie-, FDG-PET-, Scan einer Mehrzahl von Subjekten erhalten werden, wobei jedes der Mehrzahl von Bildern Informationen über ein Glucosestoffwechselmerkmal jedes der Mehrzahl von Subjekten umfasst;
Eingeben (204), über den einen oder die mehreren Prozessoren, jedes der Mehrzahl von Bildern in ein konditioniertes diffusionsbasiertes generatives Modell, um eine Mehrzahl von sequenziellen Bildern durch einen Vorwärtsdiffusionsmechanismus des konditionierten diffusionsbasierten generativen Modells zu erhalten, wobei jedes der Mehrzahl von sequenziellen Bildern Informationen über eine Ebene aus einer Mehrzahl von Ebenen eines Amyloidablagerungsmerkmals der Mehrzahl von Subjekten bereitstellt, wobei das konditionierte diffusionsbasierte generative Modell ein Codierernetzwerk, ein Decodierernetzwerk und eine Mehrschicht-Perzeptron-, MLP-, Schicht zwischen dem Codierernetzwerk und dem Decodierernetzwerk umfasst, wobei das Codierernetzwerk einen Vier-Ebenen-Codierer umfasst, wobei jede Ebene eine einzelne Faltungsschicht umfasst, die eine Kanalaufmerksamkeit verwendet, und wobei an jeder Faltungsschicht eine Zeitschritteingabe, die mit einer dichten Schicht codiert ist, bereitgestellt wird, um das Codierernetzwerk beim Interpretieren der Zeitschritteingabe zu führen;
iteratives Trainieren (206), über den einen oder die mehreren Prozessoren, des konditionierten diffusionsbasierten generativen Modells unter Verwendung einer gewichteten Kombination eines vorherigen sequenziellen Bildes aus der Mehrzahl von sequenziellen Bildern und jedes der Mehrzahl von Bildern gemäß einer Mehrzahl von subjektspezifischen Demografien und einem Subjektkrankheitsstatus, bis ein übersetztes Bild erhalten wird, wobei das übersetzte Bild eine optimale Beziehung zwischen jedem der Mehrzahl von Bildern und der Mehrzahl von Ebenen des Amyloidablagerungsmerkmals angibt; und
Vorhersagen (208), über den einen oder die mehreren Prozessoren, einer Ebene des Amyloidablagerungsmerkmals für ein eingehendes Bild, das aus dem FDG-PET-Scan eines neuen Subjekts unter Verwendung eines trainierten konditionierten diffusionsbasierten generativen Modells erhalten wird.

2. Prozessorimplementiertes Verfahren nach Anspruch 1, wobei die Mehrzahl von subjektspezifischen Demografien nach dem Codieren in die MLP-Schicht des konditionierten diffusionsbasierten generativen Modells weitergeleitet wird.

3. System (100), umfassend:
einen Speicher (102), der Anweisungen speichert;
eine oder mehrere Kommunikationsschnittstellen (106); und
einen oder mehrere Hardwareprozessoren (104), die über die eine oder die mehreren Kommunikationsschnittstellen (106) mit dem Speicher (102) gekoppelt sind, wobei der eine oder die mehreren Hardwareprozessoren (104) durch die Anweisungen konfiguriert sind zum:
Empfangen einer Mehrzahl von Bildern, die aus einem Fluordeoxyglucose-Positronen-Emissionstomographie-, FDG-PET-, Scan einer Mehrzahl von Subjekten erhalten werden, wobei jedes der Mehrzahl von Bildern Informationen über ein Glucosestoffwechselmerkmal jedes der Mehrzahl von Subjekten umfasst;
Eingeben jedes der Mehrzahl von Bildern in ein konditioniertes diffusionsbasiertes generatives Modell, um eine Mehrzahl von sequenziellen Bildern durch einen Vorwärtsdiffusionsmechanismus des konditionierten diffusionsbasierten generativen Modells zu erhalten, wobei jedes der Mehrzahl von sequenziellen Bildern Informationen über eine Ebene aus einer Mehrzahl von Ebenen eines Amyloidablagerungsmerkmals der Mehrzahl von Subjekten bereitstellt, wobei das konditionierte diffusionsbasierte generative Modell ein Codierernetzwerk, ein Decodierernetzwerk und eine Mehrschicht-Perzeptron-, MLP-, Schicht zwischen dem Codierernetzwerk und dem Decodierernetzwerk umfasst, wobei das Codierernetzwerk einen Vier-Ebenen-Codierer umfasst, wobei jede Ebene eine einzelne Faltungsschicht umfasst, die eine Kanalaufmerksamkeit verwendet, und wobei an jeder Faltungsschicht eine Zeitschritteingabe, die mit einer dichten Schicht codiert ist, bereitgestellt wird, um das Codierernetzwerk beim Interpretieren der Zeitschritteingabe zu führen;
iteratives Trainieren des konditionierten diffusionsbasierten generativen Modells unter Verwendung einer gewichteten Kombination eines vorherigen sequenziellen Bildes aus der Mehrzahl von sequenziellen Bildern und jedes der Mehrzahl von Bildern gemäß einer Mehrzahl von subjektspezifischen Demografien und einem Subjektkrankheitsstatus, bis ein übersetztes Bild erhalten wird, wobei das übersetzte Bild eine optimale Beziehung zwischen jedem der Mehrzahl von Bildern und der Mehrzahl von Ebenen des Amyloidablagerungsmerkmals angibt; und
Vorhersagen einer Ebene des Amyloidablagerungsmerkmals für ein eingehendes Bild, das aus dem FDG-PET-Scan eines neuen Subjekts unter Verwendung eines trainierten konditionierten diffusionsbasierten generativen Modells erhalten wird.

4. System (100) nach Anspruch 3, wobei die Mehrzahl von subjektspezifischen Demografien nach dem Codieren in die MLP-Schicht des konditionierten diffusionsbasierten generativen Modells weitergeleitet wird.

5. Ein oder mehrere nichtflüchtige maschinenlesbare Informationsspeichermedien, umfassend eine oder mehrere Anweisungen, die bei Ausführung durch einen oder mehrere Hardwareprozessoren bewirken:
Empfangen einer Mehrzahl von Bildern, die aus einem Fluordeoxyglucose-Positronen-Emissionstomographie-, FDG-PET-, Scan einer Mehrzahl von Subjekten erhalten werden, wobei jedes der Mehrzahl von Bildern Informationen über ein Glucosestoffwechselmerkmal jedes der Mehrzahl von Subjekten umfasst;
Eingeben jedes der Mehrzahl von Bildern in ein konditioniertes diffusionsbasiertes generatives Modell, um eine Mehrzahl von sequenziellen Bildern durch einen Vorwärtsdiffusionsmechanismus des konditionierten diffusionsbasierten generativen Modells zu erhalten, wobei jedes der Mehrzahl von sequenziellen Bildern Informationen über eine Ebene aus einer Mehrzahl von Ebenen eines Amyloidablagerungsmerkmals der Mehrzahl von Subjekten bereitstellt, wobei das konditionierte diffusionsbasierte generative Modell ein Codierernetzwerk, ein Decodierernetzwerk und eine Mehrschicht-Perzeptron-, MLP-, Schicht zwischen dem Codierernetzwerk und dem Decodierernetzwerk umfasst, wobei das Codierernetzwerk einen Vier-Ebenen-Codierer umfasst, wobei jede Ebene eine einzelne Faltungsschicht umfasst, die eine Kanalaufmerksamkeit verwendet, und wobei an jeder Faltungsschicht eine Zeitschritteingabe, die mit einer dichten Schicht codiert ist, bereitgestellt wird, um das Codierernetzwerk beim Interpretieren der Zeitschritteingabe zu führen;
iteratives Trainieren des konditionierten diffusionsbasierten generativen Modells unter Verwendung einer gewichteten Kombination eines vorherigen sequenziellen Bildes aus der Mehrzahl von sequenziellen Bildern und jedes der Mehrzahl von Bildern gemäß einer Mehrzahl von subjektspezifischen Demografien und einem Subjektkrankheitsstatus, bis ein übersetztes Bild erhalten wird, wobei das übersetzte Bild eine optimale Beziehung zwischen jedem der Mehrzahl von Bildern und der Mehrzahl von Ebenen des Amyloidablagerungsmerkmals angibt; und
Vorhersagen einer Ebene des Amyloidablagerungsmerkmals für ein eingehendes Bild, das aus dem FDG-PET-Scan eines neuen Subjekts unter Verwendung eines trainierten konditionierten diffusionsbasierten generativen Modells erhalten wird.

6. Ein oder mehrere nichtflüchtige maschinenlesbare Informationsspeichermedien nach Anspruch 5, wobei die Mehrzahl von subjektspezifischen Demografien nach dem Codieren in die MLP-Schicht des konditionierten diffusionsbasierten generativen Modells weitergeleitet wird.

## Revendications

1. Procédé mis en œuvre par processeur (200), comprenant :
la réception (202), via un ou plusieurs processeurs, d'une pluralité d'images obtenues à partir d'un balayage de tomographie par émission de positons au fluorodésoxyglucose, FDG-PET, d'une pluralité de sujets, dans lequel chacune de la pluralité d'images comprend des informations sur une caractéristique de métabolisme du glucose de chacun de la pluralité de sujets;
l'entrée (204), via les un ou plusieurs processeurs, de chacune de la pluralité d'images dans un modèle génératif basé sur la diffusion conditionnelle pour obtenir une pluralité d'images séquentielles par l'intermédiaire d'un mécanisme de diffusion vers l'avant du modèle génératif basé sur la diffusion conditionnelle, dans lequel chacune de la pluralité d'images séquentielles fournit des informations sur un niveau à partir d'une pluralité de niveaux d'une caractéristique de dépôt amyloïde de la pluralité de sujets, dans lequel le modèle génératif basé sur la diffusion conditionnelle comprend un réseau de codeur, un réseau de décodeur, et une couche perceptron multicouche, MLP, entre le réseau de codeur et le réseau de décodeur, dans lequel le réseau de codeur comprend un codeur à quatre niveaux où chaque niveau comprend une couche convolutive unique qui utilise une attention de canal, et dans lequel au niveau de chaque couche convolutive, une entrée de pas temporel codée avec une couche dense est fournie pour guider le réseau de codeur dans l'interprétation de l'entrée de pas temporel ;
l'entraînement itératif (206), via les un ou plusieurs processeurs, du modèle génératif basé sur la diffusion conditionnelle à l'aide d'une combinaison pondérée d'une image séquentielle précédente à partir de la pluralité d'images séquentielles et de chacune de la pluralité d'images conformément à une pluralité de données démographiques spécifiques au sujet et à un état pathologique du sujet jusqu'à ce qu'une image traduite soit obtenue, dans lequel l'image traduite est indicative d'une relation optimale entre chacune de la pluralité d'images et la pluralité de niveaux de la caractéristique de dépôt amyloïde ; et
la prédiction (208), via les un ou plusieurs processeurs, d'un niveau de la caractéristique de dépôt amyloïde pour une image entrante obtenue à partir du balayage FDG-PET d'un nouveau sujet à l'aide d'un modèle génératif conditionné entraîné basé sur la diffusion.

2. Procédé mis en œuvre par processeur selon la revendication 1, dans lequel la pluralité de données démographiques spécifiques au sujet sont passées dans la couche MLP du modèle génératif basé sur la diffusion conditionnelle après codage.

3. Système (100), comprenant :
une mémoire (102) stockant des instructions ;
une ou plusieurs interfaces de communication (106) ; et
un ou plusieurs processeurs matériels (104) couplés à la mémoire (102) via les une ou plusieurs interfaces de communication (106), dans lequel les un ou plusieurs processeurs matériels (104) sont configurés par les instructions pour :
recevoir une pluralité d'images obtenues à partir d'un balayage de tomographie par émission de positons au fluorodésoxyglucose, FDG-PET, d'une pluralité de sujets, dans lequel chacune de la pluralité d'images comprend des informations sur une caractéristique de métabolisme du glucose de chacun de la pluralité de sujets ;
entrer chacune de la pluralité d'images dans un modèle génératif basé sur la diffusion conditionnelle pour obtenir une pluralité d'images séquentielles par l'intermédiaire d'un mécanisme de diffusion vers l'avant du modèle génératif basé sur la diffusion conditionnelle, dans lequel chacune de la pluralité d'images séquentielles fournit des informations sur un niveau à partir d'une pluralité de niveaux d'une caractéristique de dépôt amyloïde de la pluralité de sujets, dans lequel le modèle génératif basé sur la diffusion conditionnelle comprend un réseau de codeur, un réseau de décodeur, et une couche perceptron multicouche, MLP, entre le réseau de codeur et le réseau de décodeur, dans lequel le réseau de codeur comprend un codeur à quatre niveaux où chaque niveau comprend une couche convolutive unique qui utilise une attention de canal, et dans lequel au niveau de chaque couche convolutive, une entrée de pas temporel codée avec une couche dense est fournie pour guider le réseau de codeur dans l'interprétation de l'entrée de pas temporel ;
entraîner itérativement le modèle génératif basé sur la diffusion conditionnelle à l'aide d'une combinaison pondérée d'une image séquentielle précédente à partir de la pluralité d'images séquentielles et de chacune de la pluralité d'images conformément à une pluralité de données démographiques spécifiques au sujet et à un état pathologique du sujet jusqu'à ce qu'une image traduite soit obtenue, dans lequel l'image traduite est indicative d'une relation optimale entre chacune de la pluralité d'images et la pluralité de niveaux de la caractéristique de dépôt amyloïde ; et
prédire un niveau de la caractéristique de dépôt amyloïde pour une image entrante obtenue à partir du balayage FDG-PET d'un nouveau sujet à l'aide d'un modèle génératif conditionné entraîné basé sur la diffusion.

4. Système (100) selon la revendication 3, dans lequel la pluralité de données démographiques spécifiques au sujet sont passées dans la couche MLP du modèle génératif basé sur la diffusion conditionnelle après codage.

5. Un ou plusieurs supports de stockage d'informations lisibles par machine non transitoires comprenant une ou plusieurs instructions qui, lorsqu'elles sont exécutées par un ou plusieurs processeurs matériels, amènent :
la réception d'une pluralité d'images obtenues à partir d'un balayage de tomographie par émission de positons au fluorodésoxyglucose, FDG-PET, d'une pluralité de sujets, dans lequel chacune de la pluralité d'images comprend des informations sur une caractéristique de métabolisme du glucose de chacun de la pluralité de sujets ;
l'entrée de chacune de la pluralité d'images dans un modèle génératif basé sur la diffusion conditionnelle pour obtenir une pluralité d'images séquentielles par l'intermédiaire d'un mécanisme de diffusion vers l'avant du modèle génératif basé sur la diffusion conditionnelle, dans lequel chacune de la pluralité d'images séquentielles fournit des informations sur un niveau à partir d'une pluralité de niveaux d'une caractéristique de dépôt amyloïde de la pluralité de sujets, dans lequel le modèle génératif basé sur la diffusion conditionnelle comprend un réseau de codeur, un réseau de décodeur, et une couche perceptron multicouche, MLP, entre le réseau de codeur et le réseau de décodeur, dans lequel le réseau de codeur comprend un codeur à quatre niveaux où chaque niveau comprend une couche convolutive unique qui utilise une attention de canal, et dans lequel au niveau de chaque couche convolutive, une entrée de pas temporel codée avec une couche dense est fournie pour guider le réseau de codeur dans l'interprétation de l'entrée de pas temporel ;
l'entraînement itératif du modèle génératif basé sur la diffusion conditionnelle à l'aide d'une combinaison pondérée d'une image séquentielle précédente à partir de la pluralité d'images séquentielles et de chacune de la pluralité d'images conformément à une pluralité de données démographiques spécifiques au sujet et à un état pathologique du sujet jusqu'à ce qu'une image traduite soit obtenue, dans lequel l'image traduite est indicative d'une relation optimale entre chacune de la pluralité d'images et la pluralité de niveaux de la caractéristique de dépôt amyloïde ; et
la prédiction d'un niveau de la caractéristique de dépôt amyloïde pour une image entrante obtenue à partir du balayage FDG-PET d'un nouveau sujet à l'aide d'un modèle génératif conditionné entraîné basé sur la diffusion.

6. Un ou plusieurs supports de stockage d'informations lisibles par machine non transitoires selon la revendication 5, dans lequel la pluralité de données démographiques spécifiques au sujet sont passées dans la couche MLP du modèle génératif basé sur la diffusion conditionnelle après codage.
